(19) Europäisches Patentamt
European Patent Office
Office européen des brevets

(11) **EP 3 937 954 B1**

(12) **EUROPEAN PATENT SPECIFICATION**

(45) Date of publication and mention
of the grant of the patent:
**30.04.2025 Bulletin 2025/18**

(51) International Patent Classification (IPC):
*A61K 35/12* (2015.01)     *A61K 35/16* (2015.01)
*C12N 5/078* (2010.01)

(21) Application number: **20713977.5**

(22) Date of filing: **13.03.2020**

(52) Cooperative Patent Classification (CPC):
**A61K 35/16; C12N 5/0634;** C12N 2500/84;
C12N 2501/15; C12N 2501/65; C12Q 2600/178;
G01N 2333/495

(86) International application number:
**PCT/IB2020/052286**

(87) International publication number:
**WO 2020/188433 (24.09.2020 Gazette 2020/39)**

(54) **METHOD FOR PREDICTING PROANGIOGENIC POTENTIAL OF EXTRACELLULAR VESICLES (EVS)**

VERFAHREN ZUR VORHERSAGE DES PROANGIOGENEN POTENZIALS VON EXTRAZELLULÄREN VESIKELN (EVS)

MÉTHODE DE PRÉDICTION DU POTENTIEL PROANGIOGÉNIQUE DE VÉSICULES EXTRACELLULAIRES (EVS)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2019 EP 19163194**

(43) Date of publication of application:
**19.01.2022 Bulletin 2022/03**

(73) Proprietor: **Unicyte EV AG**
**6370 Oberdorf (CH)**

(72) Inventors:
 • **BRIZZI, Maria Felice**
 **10133 Torino (IT)**
 • **CAMUSSI, Giovanni**
 **10132 Torino (IT)**
 • **CAVALLARI, Claudia**
 **10124 Torino (IT)**

(74) Representative: **Rimini, Rebecca et al**
**Jacobacci & Partners S.p.A.**
**Corso Emilia, 8**
**10152 Torino (IT)**

(56) References cited:
**WO-A1-2014/125277**     **WO-A1-2016/156865**
**WO-A1-2016/196822**     **WO-A1-2018/069408**

• **FIGLIOLINI F. ET AL.: "Isolation, characterization and potential role in beta cell-endothelium cross-talk of extracellular vesicles released from human pancreatic islets", PLOS ONE, vol. 9, no. 7, E102521, 16 July 2014 (2014-07-16), pages 1 - 14, XP055597256**
• **CAVALLARI C. ET AL.: "Serum-derived extracellular vesicles (EVs) impact on vascular remodeling and prevent muscle damage in acute hind limb ischemia", SCI. REP., vol. 7, no. 1, 8180, 15 August 2017 (2017-08-15), pages 1 - 14, XP055597703**
• **CAVALLARI C. ET AL.: "miR-130a and tgfbeta content in extracellular vesicles derived from the serum of subjects at high cardiovascular risk predicts their in-vivo angiogenic potential", SCI. REP., vol. 10, no. 1, 706, 20 January 2020 (2020-01-20), pages 1 - 13, XP055704857**

EP 3 937 954 B1

Remarks:

The complete document including Reference Table(s) and the Sequence Listing(s) can be downloaded from the EPO website

Remarks:

The complete document including Reference Table(s) and the Sequence Listing(s) can be downloaded from the EPO website

**EP 3 937 954 B1**

**Description**

The present invention relates to a method of predicting proangiogenic activity of extracellular vesicle (EV) preparations, the EV preparations thereof and their therapeutic applications.

Extracellular vesicles (EVs) are small vesicles shed from almost all cell types under both normal and pathological conditions. They mainly include microvesicles, generated by the budding of cell plasma membranes, and exosomes, derived from the endosomal membrane compartment by exocytosis. Recent evidences suggest that EVs could act as mediators of a variety of pathophysiological processes. Increased circulating EVs level has been associated with vascular impairment and hypercoagulability, in particular in patients with diabetes and acute coronary syndrome, suggesting a role in driving cardiovascular diseases. Moreover, an increased level of circulating EVs, mainly originated from platelets and endothelial cells has been proposed as hallmark of cell dysfunction. It has been extensively reported that EVs act as biological active vectors and participate in exchanging information between circulating cells and many cell types including endothelial cells. Indeed, it has been also proposed that EVs derived from platelets play a role in the pathogenesis of atherosclerosis.

EVs act as biological intermediaries mainly by delivering proteins, active lipids and extracellular RNAs, typically referred to as EVs cargo (Pathan M., et al. Vesiclepedia 2019: a compendium of RNA, proteins, lipids and metabolites in extracellular vesicles. (2019) Nucleic Acids Res. 47: D516-D519) However, the most studied EV-mediated biological processes relied on miR transfer. miRs are a class of small noncoding RNAs that post-transcriptionally regulate gene expression. miRs are stably expressed in serum/plasma and their unique expression patterns has been proposed to serve as a disease fingerprint in many clinical settings. Moreover, it has been shown that activated platelets can transfer functional miRs into vascular cells using EVs. This, on turn, regulates ICAM-1 expression and the vascular inflammatory response. Indeed, change in circulating EV cargo has been shown to be associated with endothelial and smooth muscle cell dysfunction in diabetes.

A growing body of evidence suggests that EVs may act as potential therapeutic, diagnostic, and prognostic tools.

The increased risk of cardiovascular events is a common feature of patients suffering of diabetes and obesity. The impaired vessel formation is still considered a relevant mechanism accounting for the abnormal vascular remodeling in these clinical settings. Therefore, to boost neovascularization of damaged tissues remains mandatory to improve patient's outcomes. Different therapeutic approaches have been proposed to improve vascular remodeling in patients with cardiovascular risk factors. However, they failed to provide real benefits, indicating that novel treatment options are still required

WO2018069408 discloses compositions of blood-derived EVs which are characterized by strong proangiogenic activity. Moreover, WO2018069408 teaches a test enabling to measure the proangiogenic potential of EVs, which includes assaying the EVs for their ability to induce cell proliferation and/or a tube-like structure formation in vitro.

The present invention is based on the finding that the composition of the EVs cargo represents a reliable predictive indicator as to whether these vesicles exhibit proangiogenic activity. Surprisingly, the experimental studies carried out by the present inventors, which are illustrated in detail in the experimental section that follows, revealed that EVs possessing proangiogenic activity may be distinguished from inactive vesicles based on the content of transforming growth factor beta (TGFβ) used in combination with the measurement of miR-130a. Indeed, as deduced from ROC analysis, a test based on the combination of the aforementioned measurements exhibits a strong predictive power of discrimination for EVs proangiogenic activity in terms of both sensitivity and specificity, thereby enabling the accurate selection of EVs which are effective in the therapeutic treatment of a disease or injury positively influenced by proangiogenic therapy.

Accordingly, a first aspect of the present invention is a method of predicting whether a composition of extracellular vesicles (EVs) has proangiogenic activity, comprising the steps of:

(a) quantifying the miR-130a microRNA content in the composition of EVs, and
(b) quantifying the transforming growth factor beta (TGFβ) content in the composition of EVs;
(c) determining whether the miR-130a content is above a first predetermined value and the TGFβ content is above a second predetermined value,

wherein:

when the miR-130a content is above said first predetermined value and the TGFβ content is above said second predetermined value, the composition of EVs is predicted to have proangiogenic activity.

As used herein, the term "proangiogenic activity" refers to the stimulation or enhancement of angiogenesis and/or endothelial cell proliferation.

As is further explained in detail below, the inventors have conducted a microarray-based expression profiling of microRNAs (miRNAs) in proangiogenic effective and ineffective EVs isolated from the blood of healthy donors and patients with cardiovascular risk factors, and surprisingly found a significant correlation between the proangiogenic activity of these vesicles and the content of miR-130a. Further studies revealed that EVs possessing proangiogenic activity contain higher amount of the TGFβ protein, as compared to non-active EVs.

Without wishing to be bound by any theory, the inventors believe that the role played by miR-130a in promoting the angiogenic process may be explained by the interaction of this molecule with several genes involved in the angiogenic process, such as KDR, HOXA5, ROCK1, and EPHB6 as revealed by the IPA Ingenuity bioinformatic analysis conducted by the inventors. Moreover, as a result of this analysis, TGFβ and TGFBR1 were also found among the genes under the control of miR-130a, further confirming the cooperation between miR-130a and TGFβ in driving the proangiogenic activity of biologically active EVs.

According to the present invention, the miR-130a nucleotide sequence comprises or consists of nucleotide sequence 5' -CAGUGCAAUGUUAAAAGGGCAU -3' (SEQ ID NO. 1).

In the method according to the invention, the quantification of miR-130a in the EVs composition is preferably carried out by a nucleic acid-based amplification technique, more preferably by real-time PCR.

In a preferred embodiment, the miR-130 content assessed in the composition of EVs by real-time PCR is measured as a threshold cycle (Ct) value.

Typically, nucleic acid quantification by real-time PCR relies on plotting amplification signal, for example fluorescence, against the number of cycles on a logarithmic scale. As used herein, the term "Ct value" refers to the number of PCR cycles required for the amplification signal to reach an intensity above the background level during the exponential phase of the nucleic acid amplification reaction. Consequently, the Ct value is inversely related to the amount of target nucleic acid initially present in the sample, i.e. the greater the abundance of the target nucleic acid, the smaller the Ct value. Methods for determining the background level in a real-time PCR reaction are well established and known to the person skilled in the art.

More particularly, the present inventors observed that a content of miR130a measured as Ct value of Ct > 30 in serum EVs (sEVs) both from healthy subjects and from patients is predictive of proangiogenic ineffective vesicles (< 50% as measured in an in vitro potency test).

Accordingly, the EVs composition in the method of the invention is determined to have a miR130a content above a predetermined value. Preferably, the EVs composition in the method of the invention is determined to have a miR130a content as measured as Ct value of Ct less than or equal to 35, more preferably of Ct less than or equal to 33, still more preferably of Ct less than or equal to 30, even more preferably of Ct comprised within the range of from 10 to 29 such as for example 10, 11, 12, 13, 14, 15, 15, 16, 17, 18, 19, 20, 21, 22, 23, 24, 25, 26, 27, 28 or 29.

In a preferred embodiment, the EVs composition in the method of the invention is determined to have a miR130a content as measured as Ct value of Ct < 30.

In a preferred embodiment, the amount of miR-130a content in the EVs is determined as a Ct value by applying the $2^{-(\Delta Ct)}$ method, which is based on a formula which allows to calculate the relative fold gene expression of samples as described in Livak KJ and Schmittgen TD, "Analysis of relative gene expression data using real-time quantitative PCR and the 2(- Delta Delta C(T)) method" (2001) Methods 25: 402-408. As it will be illustrated in detail in the following examples, in order to use the delta-delta Ct method, Ct values measured for all the genes examined and the housekeeping genes have been taken into consideration by the present inventors. Because the whole analysis was based on different housekeeping genes, it was previously made the average of all the housekeeping genes Ct values (Human U& snRNA, RNU43 snoRNA, Hm/Ms/RT U1 snRNA).

In an another preferred embodiment, the quantification of miR-130a content in the EVs is achieved by employing a standard curve of known amounts of miR-130a and interpolating the Ct value determined by real-time PCR in the unknown sample with the standard curve.

While reference is made to real-time PCR, it is understood that other methods of nucleic acid amplification may be used within the scope of this invention, as are known in the art. Such methods include, but are not limited to, nucleic acid sequence-based amplification (NASBA) and digital PCR.

With their studies, the present inventors found that sEVs both from healthy subjects and from patients, which have a content of TGFβ below a predetermined value, preferably below 23 pg/$10^{10}$ EVs, are predicted to be proangiogenic inactive.

Therefore, the EVs composition in the method of the invention is determined to have a content of TGFβ above a predetermined value, preferably above a value comprised within the range of from 20 pg/$10^{10}$ EVs to 50 pg/$10^{10}$ EVs, more preferably above a value within the range of from 23 pg/$10^{10}$ EVs to 40 pg/$10^{10}$ EVs, even more preferably above a value within the range of from 25 pg/$10^{10}$ EVs to 35 pg/$10^{10}$ EVs, such as for example 25, 26, 27, 28, 29, 30, 31, 32, 33, 34 or 35 pg/$10^{10}$ EVs.

In a preferred embodiment, the EVs composition in the method of the invention is determined to have a content of TGFβ of at least 23 pg/$10^{10}$ EVs.

According to the method of the invention, the quantification of TGFβ protein in EVs may be carried out in any suitable manner such as those known in the protein field.

Preferably, the TGFβ content of EVs is measured by an immunoassay. Any suitable immunoassay may be employed, for example, enzyme-linked immunosorbent assay (ELISA), chemiluminescence immunoassay (CLIA), fluorescent immunoassay (FIA), radioimmunoassay (RIAs), precipitation immunoassays, particle immunoassays, competitive binding assays, and the like. More preferably, the immunoassay employed in the method of the invention is an ELISA assay. Obviously, the use of any type of immunoassay format, the selection of which falls within the skills of the ordinary person of skill in the art, is within the scope of the present invention.

In all of the above-described embodiments, it is also preferred that the EVs are derived from human cells.

According to one embodiment of the method of the invention, the proangiogenic activity of the composition of EVs is quantified with an in vitro potency test, which involves testing the EVs by means of a BrdU cell proliferation assay, or a tubulogenesis in vitro assay, or both a BrdU cell proliferation assay and a tubulogenesis in vitro assay.

In a preferred embodiment, the potency test comprises the following steps:

- measuring the activity of the EVs composition by a BrdU cell proliferation assay;
- measuring the activity of a negative control by a BrdU cell proliferation assay;
- measuring the activity of a positive control by a BrdU cell proliferation assay;
- calculating the % activity of the composition in the BrdU cell proliferation assay by applying the following formula:

$$\% \, activity = \frac{composition \, value - negative \, control \, value}{positive \, crtl \, value - negative \, control \, value} \, x \, 100.$$

According to this embodiment, the method of the invention further comprises step (d) of quantifying the proangiogenic activity of the EVs composition by means of a potency test which comprises the following steps:

- testing the composition of EVs by a BrdU cell proliferation assay to obtain a composition value;
- testing a negative control by a BrdU cell proliferation assay to obtain a negative control value;
- testing a positive control by a BrdU cell proliferation assay to obtain a positive control value;
- calculating the % proangiogenic activity of the composition of EVs in the BrdU cell proliferation assay by applying the following formula:

$$\% \, proangiogenic \, activity = \frac{composition \, value - negative \, control \, value}{positive \, crtl \, value - negative \, control \, value} \, x \, 100.$$

The BrdU assay preferably uses HMEC cells seeded in matrigel. In the BrdU cell proliferation assay, serum (preferably 10% serum) is added in the positive control. The negative control is the same medium as the positive control but without the addition of serum. The BrdU assay is preferably based on a concentration of 10000 EVs per target cell.

In another preferred embodiment, the potency test comprises the following steps:

- measuring the activity of the EVs composition by a tubulogenesis assay;
- measuring the activity of a negative control by a tubulogenesis assay;
- measuring the activity of a positive control by a tubulogenesis assay;
- calculating the % activity of the composition in the tubulogenesis assay by applying the following formula:

$$\% \, activity = \frac{composition \, value - negative \, control \, value}{positive \, crtl \, value - negative \, control \, value} \, x \, 100.$$

According to this embodiment, the method of the invention further comprises step (d) of quantifying the proangiogenic activity of the EVs composition by means of a potency test which comprises the following steps:

- testing the composition of EVs by a tubulogenesis assay to obtain a composition value;
- testing a negative control by a tubulogenesis assay to obtain a negative control value;
- testing a positive control by a tubulogenesis assay to obtain a positive control value;
- calculating the % proangiogenic activity of the composition of EVs in the tubulogenesis assay by applying the following formula:

$$\% \ proangiogenic \ activity = \frac{composition \ value - negative \ control \ value}{positive \ crtl \ value - negative \ control \ value} \ x \ 100.$$

The tubulogenesis in vitro assay preferably uses HUVEC cells. In the tubulogenesis in vitro assay, VEGF, preferably 10 ng/ml VEGF, is added to the positive control. The negative control is the same medium as the positive control but without VEGF addition. The tubulogenesis in vitro assay is preferably based on a concentration of 50000 EVs per target cell.

In a more preferred embodiment, both the BrdU cell proliferation assay and the tubulogenesis in vitro assay are used for testing the activity of a given EVs composition against the activity of a positive control, in which case the average % activity values from the BrdU cell proliferation assay and the tubulogenesis assay are compared.

According to this embodiment, the method of the invention further comprises step (d) of quantifying the proangiogenic activity of the composition of EVs by means of a potency test comprising both the BrdU cell proliferation assay and the tubulogenesis in vitro assay as above described.

If the EVs measured activity exceeds a predetermined percentage of the positive control measured activity, for example >50% of the positive control measured activity, the EVs preparation tested is considered as active.

Accordingly, the EVs composition in the method of the invention is preferably determined to have a proangiogenic activity of at least 50%.

Furthermore, the above-described predictive test is suitable to screen for EVs isolated from multiple preparations of body fluid or from the conditioned medium of a cell culture and to identify the pro-angiogenically active preparations for further processing.

Thus, a second aspect of the present invention is a method of manufacturing a preparation of proangiogenic extracellular vesicles (EVs), comprising the steps of:

- isolating EVs from multiple preparations of a body fluid or from the conditioned medium of a cell culture;
- preparing one or more samples from the isolated EVs at a predetermined concentration of EVs;
- predicting the proangiogenic activity of each EVs sample with a method as above described;
- selecting the samples wherein miR-130a content is above said first predetermined value, and TGFβ is above said second predetermined value; and optionally
- pooling two or more of the active EVs samples, thereby obtaining a preparation of proangiogenic EVs.

EVs derived from serum or other blood components of healthy donors or patients with cardiovascular risk factors are able to induce proangiogenic signals in vitro and in vivo and this effect is not lost even when sEV are pooled.

As mentioned above, with their studies the inventors found that EVs compositions determined to have a content of miR-130a and TGFβ above a first and second predetermined value, respectively, are predicted to possess strong pro-angiogenic properties. These features make them particularly suitable to be employed in the treatment of ischemic diseases, ischemic injuries and pathological conditions associated with risk of cardiovascular disease, or for use in wound healing.

Accordingly, a third aspect of the present invention is a preparation of proangiogenic extracellular vesicles (EVs) wherein the miR-130a content in the preparation measured as Ct value by real-time PCR is Ct < 30 and the TGFβ content is > 23 pg/$10^{10}$ EVs, for use in the therapeutic treatment of a disease or injury positively influenced by proangiogenic therapy or for use in wound healing.

The preparation of proangiogenic extracellular vesicles (EVs) for use according to the invention as above defined, is obtainable by the aforementioned method of manufacturing.

The condition associated with risk of cardiovascular disease treated with the EVs preparation according to the present invention is preferably characterized by impaired vascular remodeling, more preferably is obesity, diabetes mellitus, dyslipidemia or hypertension.

In a preferred embodiment, the EVs preparation according to the present invention is suitable for use in the treatment of a disease selected from the group consisting of acute myocardial infarction, acute cerebrovascular disease, acute and chronic peripheral artery disease, acute kidney ischemia, obesity and diabetes mellitus.

Extracellular vesicles are produced by many different cell types - so-called donor cells - and are ubiquitously present in biological fluids, and cellular or tissue cultures. Thus, in accordance with the present invention, the composition of EVs can be obtained from any suitable cell type, preferably from a nucleated mammalian cell, more preferably from a stem cell, even more preferably from an adult stem cell.

Within the context of the present description, the expression "adult stem cell" is intended to mean a stem cell that is isolated from an adult tissue, in contrast with an "embryonic stem cell" which is isolated from the inner cell mass of a blastocyst. Adult stem cells are also known as "somatic stem cells".

According to an alternative embodiment, the EVs preparation for use according to the invention is isolated from a biological fluid or from a conditioned cell or tissue culture medium.

Preferably, the EVs preparation for use according to the invention is isolated from a blood component, more preferably whole blood, plasma or serum (sEV).

In one embodiment, the proangiogenic EVs are prepared from a blood donation of a healthy donor.

In another embodiment, the proangiogenic EVs are prepared from a blood donation of a patient, more preferably from a patient with cardiovascular risk factors.

In a more preferred embodiment, the proangiogenic EVs prepared from the blood of a patient with cardiovascular risk factors are suitable for use as a medicament for the treatment of the same patient.

Furthermore, in all of the above-described embodiments, the preparation of the invention may be a pharmaceutical preparation, which includes proangiogenic EVs as defined above as well as a pharmaceutically acceptable excipient and/or carrier and/or diluent. The selection of the carrier, vehicle or diluent as well as of any other excipient falls within the skills of the person skilled in the art taking into account, inter alia, the selected pharmaceutical dosage form, administration route and administration regimen, as well as the patient's characteristics and the disease to be treated.

The invention will be better understood from the following examples which are provided by way of illustration only and which make reference to the appended drawings, wherein:

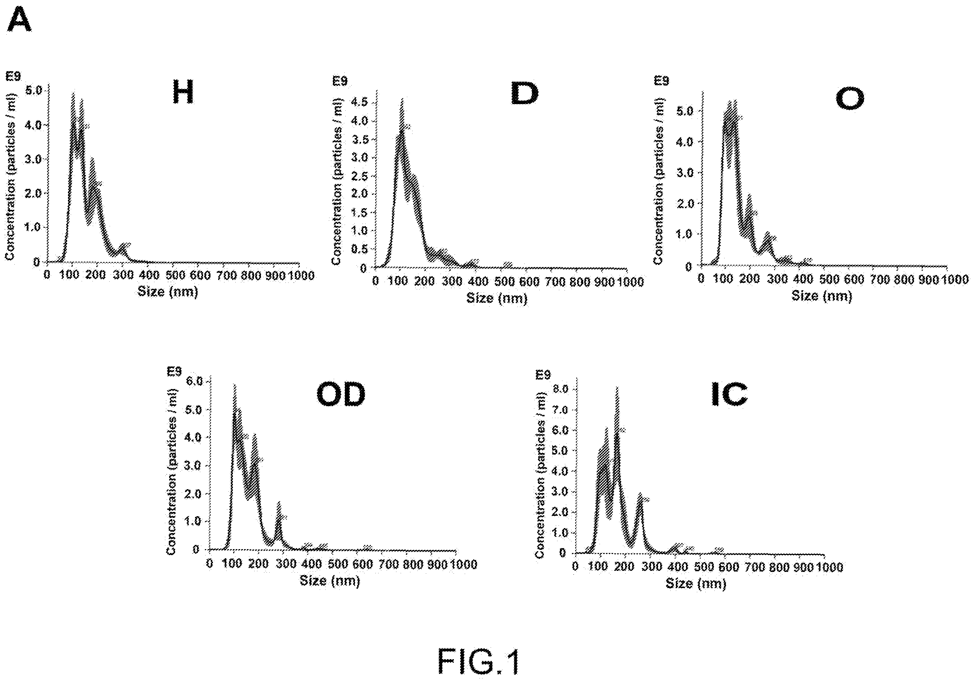

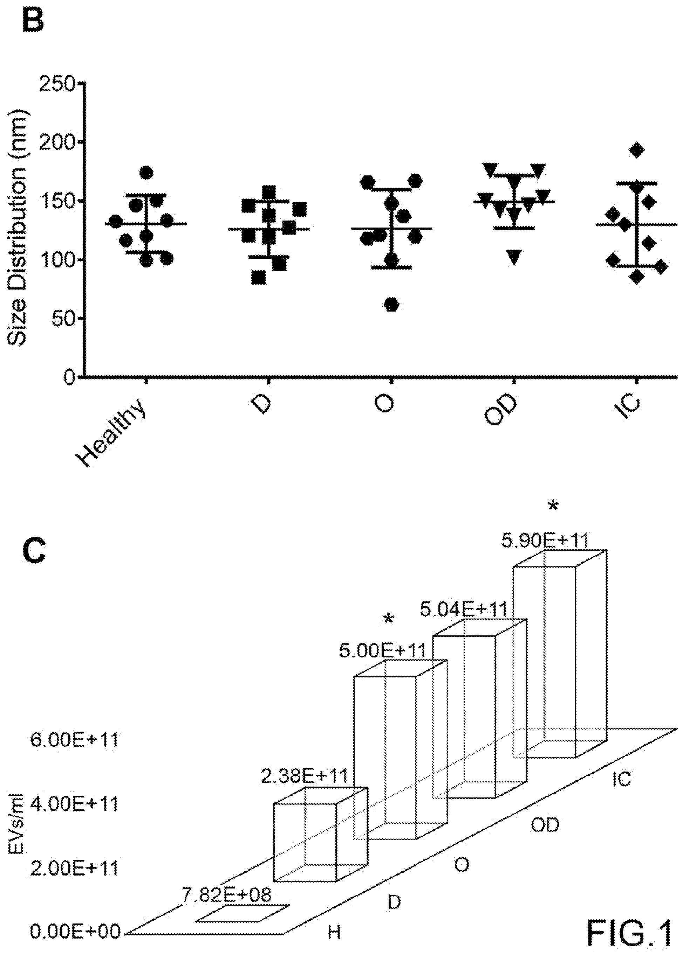

**Figure 1** shows the results of sEV characterization by Nanosight. (A) Representative images of NTA analysis referred to individual groups of patients. (B) Dot plot graph representing NTA size distribution, with mean size value for each individual subject (healthy donor, obese, diabetic, diabetic/obese and ischemic patient). (C) Histogram reporting the number of EVs recovered from serum from individual groups of patients. D= diabetic; O= Obese; OD= obese/diabetic; IC= Ischemic patients. *p < 0.05 obese and ischemic patients vs healthy subjects; (One-way ANOVA followed by Multiple Comparison Test) (n= 9 patients/group).

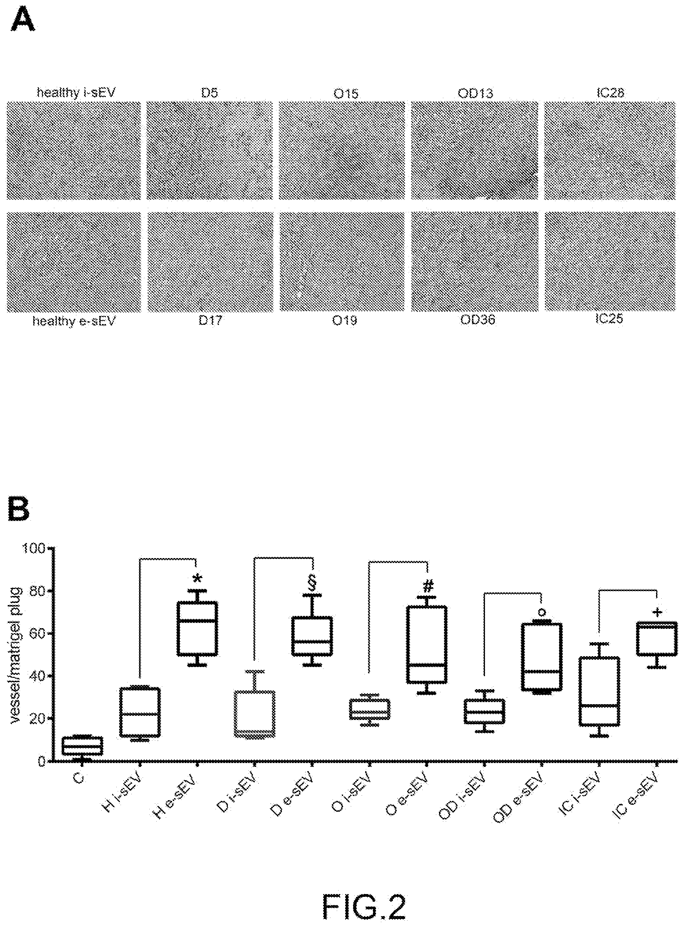

**Figure 2** shows the *in vitro* and *in vivo* pro-angiogenic activity of serum EVs from healthy donors and patients (A) Representative micrographs showing vessels formation in response to effective and ineffective sEVs. Each number refers to sEVs prepared from an individual subject (upper panel = ineffective sEVs; lower panel = effective sEVs) (n=3 each group, except for OD the same sample was used in 3 independent experiments). (B) Results of *in vivo* quantitative analysis of vessel formation. For each experimental condition, vessels were counted in 10 sections of Matrigel. Data show the average number of vessels counted in untreated mice (C) (n= 3) or in mice treated with the following preparations of EVs: proangiogenic ineffective sEVs from healthy donors (i-sEVs), proangiogenic effective sEVs from healthy donors (e-sEVs); proangiogenic ineffective sEVs from diabetic patients (D i-sEVs), proangiogenic effective sEVs from diabetic patients (D e-sEVs); proangiogenic ineffective sEVs from obese patients (O i-sEVs), proangiogenic effective sEVs from obese patients (O e-sEVs); proangiogenic ineffective sEVs from diabetic/obese patients (OD i-sEVs), proangiogenic effective sEVs from diabetic/obese patients (OD e-sEVs); proangiogenic ineffective sEVs from ischemic patients (IC i-sEVs), proangiogenic effective sEVs from ischemic patients (IC e-sEVs). *p < 0.05 healthy e-sEV vs. healthy i-sEV; §p < 0.05 diabetic e-sEV vs. diabetic i-sEV; #p < 0.05 obese e-sEV vs. obese i-sEV; °p < 0.05 diabetic/obese e-sEV vs. diabetic/obese i-sEV; +p < 0.05 ischemic e-sEV vs. ischemic i-sEV ischemic; (One-way ANOVA followed by Multiple Comparison Test). (n=3 each group except for OD the same sample was used in 3 independent experiments). (Original magnification: x200; scale bar: 12 $\mu$m).

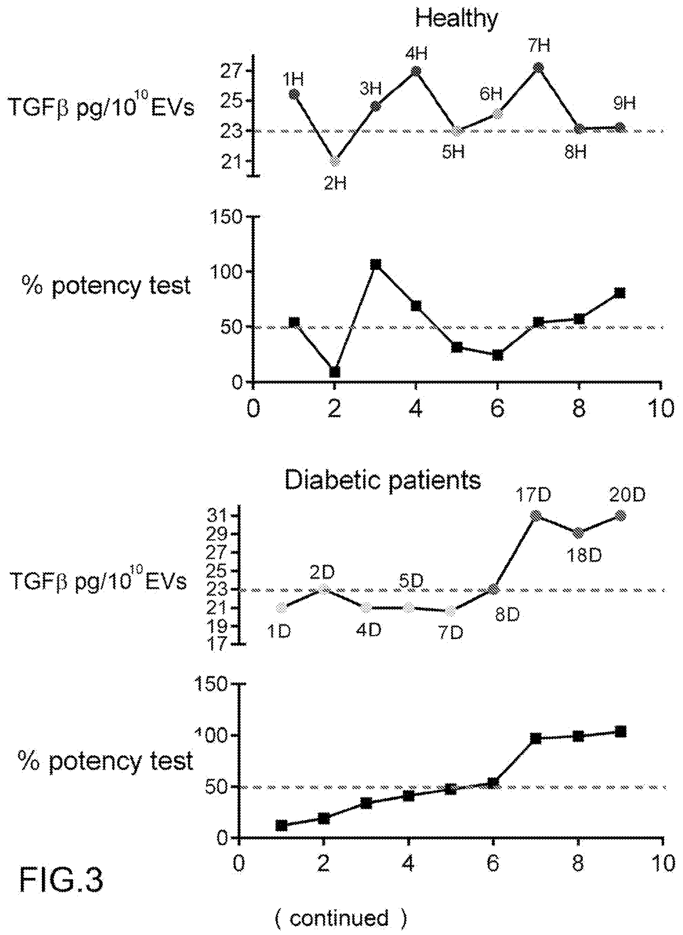

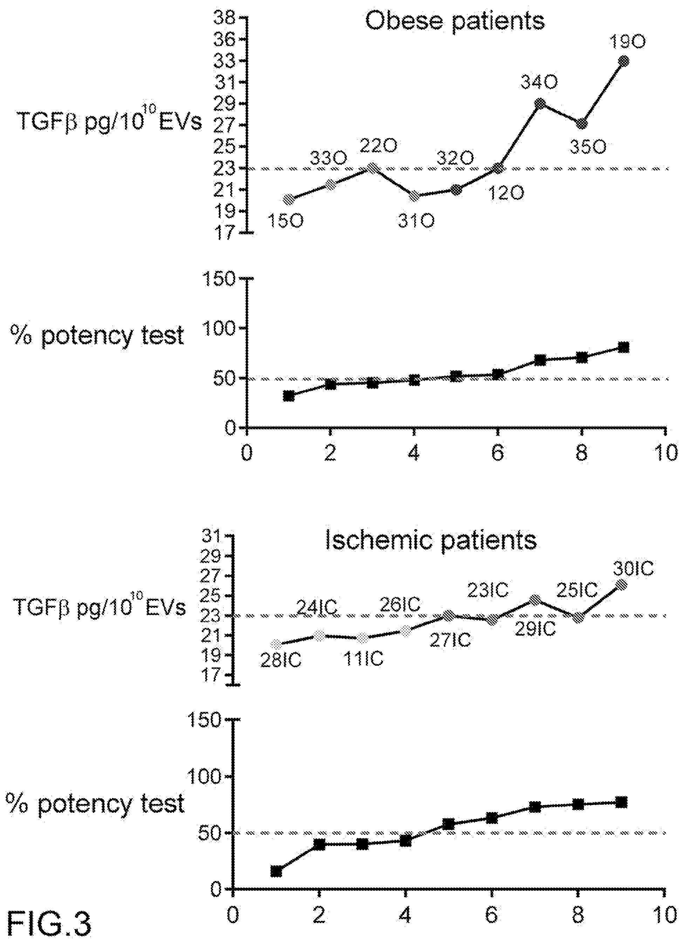

**Figure 3** shows that the proangiogenic activity of sEVs correlates with their TGF$\beta$ content. The graphs report the data obtained for samples of serum EVs prepared from individual subjects in each group (healthy donors, diabetic, obese and ischemic patients). For each group of patients, the upper curve is referred to the TGF$\beta$ content measured in sEVs as pg/$10^{10}$ EVs, while the lower curve is referred to the % of proangiogenic activity as measured in the in vitro potency test. The dotted line indicates the cut-off of TGF$\beta$ > 23 pg/$10^{10}$ EVs for proangiogenic effective and ineffective sEVs. Each number corresponds to an individual patient (n=3 each group).

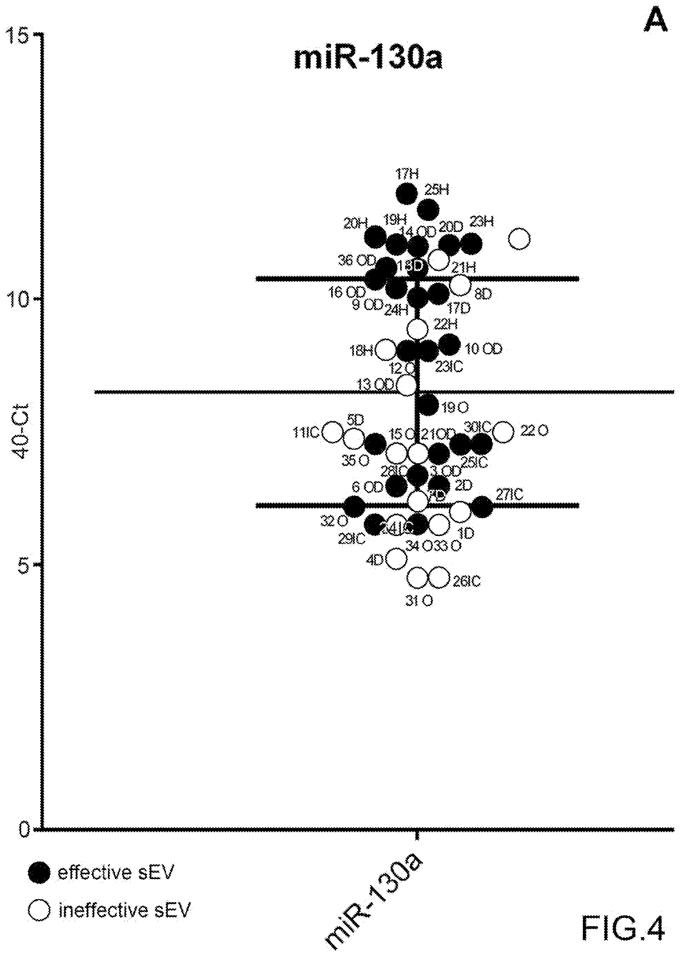

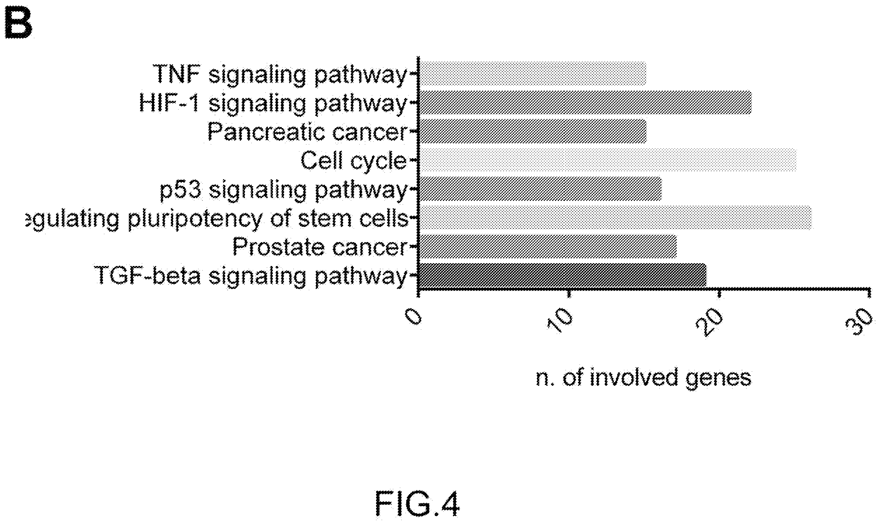

**Figure 4** shows the results of miRNAs expression profiling in sEVs. (A) Distribution of Ct values measured for miR-130a in proangiogenic effective (dark circles) and ineffective (white circles) sEVs from individual patients and healthy subjects. Results are reported as 40-Ct. (B) Network analysis of pathways positively correlated with miR-130a. Data were obtained by DIANA miRpath analysis. Only pathways including at least 15 genes were selected.

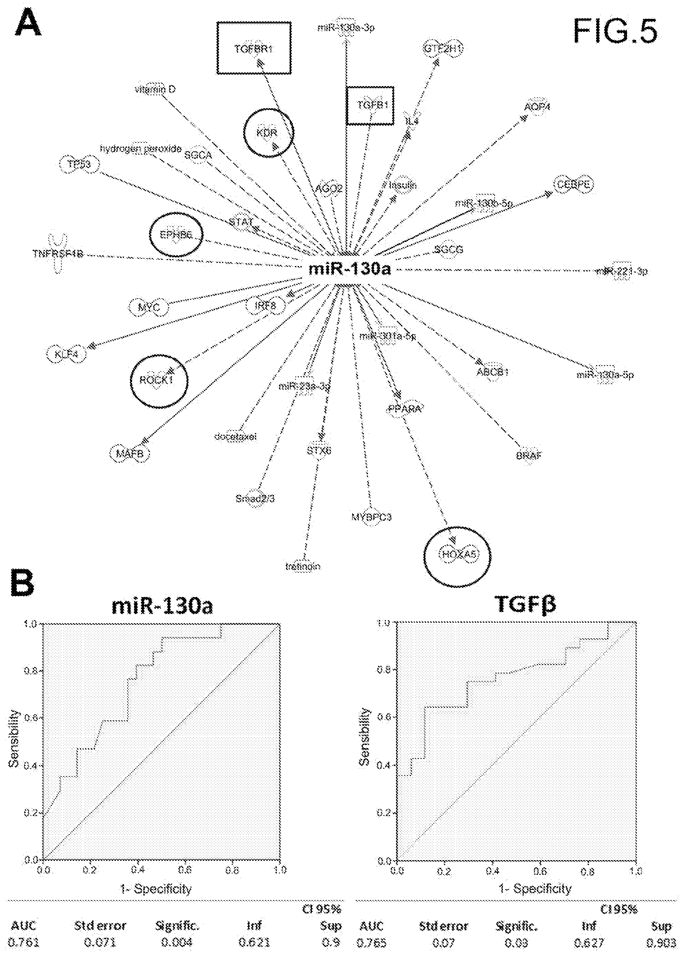

**Figure 5** (A) Network analysis between miR-130a and mRNA targets. Lines represent interactions between genes and miR-130a predicted by the IPA Software: indirect interactions (dotted lines), direct interactions (continuous lines). Squares include TGF$\beta$ and TGFBR. Circles include genes involved in angiogenesis (KDR, EPHB6, ROCK1, HOXA5). (B) Receiving Operating Characteristic (ROC) curves and the corresponding area under the curve (AUC) show that miR-130a and TGF$\beta$ have predictive ability to discriminate proangiogenic effective sEVs from ineffective vesicles. For ROC analysis, the results obtained for sEVs from all patients and healthy subjects were considered. The AUC values as well as standard errors, p-values, and threshold values are reported in the tables below the ROC curves.

## EXAMPLES

### *1. Method*

### I.1 Patients

In the study carried out by the present inventors, thirty-six patients were included with cardiovascular risk factors and nine sex-matched healthy volunteers. In particular, nine diabetic patients (D: n=9), nine obese patients (O: n=9), nine diabetic and obese patients (OD: n=9), and nine ischemic patients (patients undergoing to surgical treatment for hind limb ischemia) (IC: n=9) were examined. All diabetic patients were not treated with insulin. All human experiments were performed in accordance with European Guidelines and policies and approved by the Ethical Committee of the University of Turin, Italy. Serum from all patients was obtained after admission to the Clinics (D, O, OD) and before surgery for ischemic patients (IC). Informed consent was obtained from all patients. Human serum from healthy donors (n=9) was provided by the Blood Bank of "Città della Salute e della Scienza di Torino", after informed consent and approval by the internal Review Board of the Blood Bank.

### 1.2. Study approval

Animal studies were conducted in accordance with the Italian National Institute of Health Guide for the Care and Use of Laboratory Animals (protocol no: 490/2105-PR). Mice were housed according with the Federation of European Laboratory Animal Science Association Guidelines and the Ethical Committee of the University of Turin. All experiments were performed in accordance with relevant guidelines and regulations.

### 1.3. Serum EVs isolation

Human blood was obtained from healthy and patients donors by venipuncture. A total of 9 ml serum each donor were recovered from each donor and stored at -80 °C. After thawing, total EVs were isolated and purified by Ultracentrifugation at 100,000 $\times$g for 2 h preceded by a centrifugation at 3000g to remove debris. Pellets were washed once with PBS and centrifuged at 100.000$\times$g, 4°C for 1h. Samples were used fresh or thawed after being stored at -80°.

### 1.4 Nanoparticle tracking analysis

sEVs were analyzed by nanoparticle tracking analysis (NTA), using the NanoSight LM10 system (NanoSight Ltd., Amesbury, UK), equipped with a 405 nm laser and with the NTA 2.3 analytic software, to define their dimension and profile. All acquisitions were done with Camera level setting at 14 and for each sample, three videos of 30 s duration were recorded. sEVs were diluted (1: 1000) in 1 ml vesicle free physiologic solution (Fresenius Kabi, Runcorn, UK). NTA post-acquisition settings were optimized and maintained constant across samples, and each video was then analyzed to measure EV size, distribution and concentration.

### 1.5 sEVs angiogenic assay

Primary macrovascular endothelial cells (ECs) and microvascular endothelial cells (HMEC) were purchased from Lonza (Basel, Switzerland) and cultured as described by the manufacturer's instructions. The in vitro angiogenesis potency test and the in vivo angiogenesis test were performed as previously described (Cavallari C. et al, "Serum-derived extracellular vesicles (EVs) impact on vascular remodeling and prevent muscle damage in acute hind limb ischemia" (2017) Sci Rep. 7(1):8180). Briefly, $5\times10^4$ sEVs/target cells were administered throughout the in vitro study. sEVs from single samples were evaluated for their pro-angiogenic activity using BrdU and in vitro tubulogenesis assays. EVs of all the analyzed groups were classified as proangiogenic active or inactive EVs according to a % cut-off value of 50%.

In vivo angiogenesis was assessed by measuring the growth of blood vessels as previously described (Lopatina T. et al, "Platelet-derived growth factor regulates the secretion of extracellular vesicles by adipose mesenchymal stem cells and enhances their angiogenic potential" (2014) Cell Commun Signal. 12:26). Briefly, ECs ($1\times10^6$ cells/injection) were incubated overnight with sEVs ($5\times10^{10}$ EVs per $1\times10^6$ of ECs). Male severe combined immunodeficiency (SCID) mice (6 weeks old) were then injected subcutaneously. An equal number of non-stimulated ECs was used as a negative control. The Matrigel plugs were recovered on day 7 and fixed and stained using the trichrome stain method. The vessel lumen area was determined as previously described (Lopatina T. et al, "Platelet-derived growth factor regulates the secretion of extracellular vesicles by adipose mesenchymal stem cells and enhances their angiogenic potential" (2014) Cell Commun Signal. 12:26).

### 1.6 TGFβ ELISA assay

The TGFβ content in the EVs isolated form serum samples of healthy subjects and patients was measured using a solid phase sandwich Enzyme Linked-Immuno-Sorbent Assay (ELISA, Invitrogen Multispecies TGF-β1 kit, Germany) according to the manufacturer's instructions. Experiments were done in triplicate on samples containing $1 \times 10^{11}$ EVs. The intensity of the colored product obtained in the assay was determined with an ELISA iMarkTM Microplate Absorbance Reader (Bio Rad, Switzerland) with absorbance at 450 nm. The concentration of TGFβ present in the EVs preparations was expressed as $pg/10^{10}$ EVs.

### 1.7 miRNA expression profiling

The expression profiles of the miRNAs contained in sEVs (so-called miRNome) was assessed by real-time PCR on 1140 microRNAs using miRNome microRNA Profilers QuantiMir (SBI, System Biosciences), according to the protocol recommended by the manufacturer. The kit includes assays in pre-formatted plates for human microRNAs with three endogenous reference RNA as normalization signals (human U6 snRNA, small nucleolar RNA RNU43 and Hm/Ms/Rt U1 snRNA) on each plate.

In brief, 100 ng of RNA has been retrotranscribed using High-Capacity cDNA Reverse Transcription Kit (Applied Biosystems, Foster City, California, USA). All qRT-PCR reactions were conducted on the StepOnePlus™ Real-Time PCR System under the following conditions: 15° at 95°C (PCR Initial activation step) followed by 3-step cycling (15" at 94°C, 30'' at 55°C, 30" at 70°C) for 40 cycles. In the screening, the miRNome was profiled on sEVs collected from serum of healthy subjects, which had been assessed as proangiogenic active (n= 3) and proangiogenic inactive (n=3) with the above described potency test. The Ct values for the miRNAs were extrapolated for each sEVs sample analyzed. A Ct representing the average of Cts from different samples (n= 3) of both effective and ineffective sEVs populations was normalized against the endogenous reference RNAs and converted in $2^{-(\Delta Ct)}$ values (Livak KJ and Schmittgen TD, "Analysis of relative gene expression data using real- time quantitative PCR and the 2(- Delta Delta C(T)) method" (2001) Methods 25: 402-408).

The miRNAs validation was performed on sEVs from healthy donors and patients using the miScript SYBR Green PCR Kit (Qiagen, Valencia, CA, USA). Briefly, 100 ng of input RNA isolated from sEVs samples were reverse transcribed using the miScript Reverse Transcription Kit and the cDNA thus obtained was used to detect and quantify the miRNAs of interest. Experiments were run in triplicate using 3 ng of cDNA each reaction, as described by the manufacturer's protocol (Qiagen). The following miRNAs were screened in all patient-derived sEVs: miR-126 (SEQ ID NO. 2), miR-21 (SEQ ID NO. 3), miR-296-3p (SEQ ID NO. 4), miR-210 (SEQ ID NO. 5), miR-130a (SEQ ID NO. 1), miR-27a (SEQ ID NO. 6), miR-29a (SEQ ID NO. 7), miR-191 (SEQ ID NO. 8). The amplification data obtained with qRT-PCR were normalized using the RNU6B and the RNU43 reference genes as internal controls. The amplification efficiencies of the target sequence and the endogenous controls were shown to be approximately equal.

### 1.8 Pathway and target prediction analysis of miRNAs EV content

In order to perform EV miRNAs target prediction and biological pathway enrichment analysis, the web-based program DIANAmirPath was used (Collino F. et al, "Exosome and Microvesicle-Enriched Fractions Isolated from Mesenchymal Stem Cells by Gradient Separation Showed Different Molecular Signatures and Functions on Renal Tubular Epithelial Cells" (2017) Stem Cell Rev.(2):226-43). The algorithm microT-CDS was chosen to predict EV-derived miRNA targets using default threshold (microT = 0.8). Only biological pathways showing P value < 0.01 to all known Kyoto Encyclopedia of Genes and Genomes (KEGG) pathways wereconsidered as significantly enriched. Ingenuity IPA pathway analysis was used to predict target genes for miR-130a. The inventors set up the miRNA Target Filter tool on IPA (Qiagen: http://www.qiagen-bioinformatics.com/products/ingenuity-pathway-analysis/) to associate miR-130a with predicted mRNA targets.

### 1.9 ROC analysis

Principal data are presented as means, standard deviations (SD), median and 95% confidence intervals for the two investigated groups "True proangiogenic active sEVs"/"True proangiogenic inactive sEVs", considered as Reference Standard (RS). In order to evaluate the predictability for miR-130a and TGFβ, the achievement of RS was evaluated using ROC curves (Grund B and Sabin C. "Analysis of biomarker data: logs, odds ratios, and receiver operating characteristic curves" (2010) Curr Opin HIV AIDS 5(6):473-9. The sEVs compositions were classified into the following categories based on the content of miR-130a measured as Ct value and the content of TGFβ, measured as $pg/10^{10}$ EVs:

1. sEVs displaying a miR-130a Ct value $\geq$ 30 were considered proangiogenic ineffective sEVs;

2. sEVs displaying a TGF$\beta$ content < 23 pg/$10^{10}$ EVs were considered proangiogenic ineffective sEVs.

In order to evaluate the 'goodness' of the cut-off score based on ROC curve analysis for the above measures to predict "True proangiogenic inactive sEVs" defined in RS, the predictive capacity was evaluated both for each of the two measures separately and by combining the two measures by a 'Series' approach (considering as "proangiogenic ineffective sEVs" the sEVs which are proangiogenic ineffective for both measurements and as "NON proangiogenic ineffective sEVs" the vesicles which are "NON proangiogenic ineffective sEVs" for at least one of the two measures).

The analysis was based on Sensitivity (Se), Specificity (Sp), and positive Likelihood ratio (LH+) [Probability of identifying as "proangiogenic ineffective sEVs" a "true proangiogenic ineffective sEVs" compared to a "true proangiogenic effective sEVs"] and relative 95% Confidence Intervals values.

## 1.10 Statistical Analysis

Data were analyzed using the GraphPad Prism 6.0 Demo program. Results are expressed as mean $\pm$ SD or $\pm$ SEM, unless otherwise reported. Statistical analysis was carried out using 1-way ANOVA, followed by Tukey's post hoc or multiple comparison, Student t tests for 2-group comparison and Newman-Keuls Multiple Comparison Test where appropriate. The cut-off for statistical significance was set at p<0.05 (*p<0.05, **p<0.01, ***p<0.001).

## 2. Results

### 2.1 Characterization of serum EVs

In the study conducted by the present inventors, nine samples of sEVs derived from healthy individuals and 36 samples of sEVs derived from patients cohorts were examined for their number and size. The distribution of sEVs size did not show any significant difference among healthy individuals and patients (Figure 1A and B). The observed average particle size was around 138 nm. The sEVs number in patients was higher than in healthy subjects (Figure 1C). Significant higher levels of sEVs were detected in obese and ischemic patients (Figure 1C).

### 2.2 Pro-angiogenic activity of serum EVs derived from patients

In order to evaluate in vitro the angiogenic activity of sEVs derived from different patient's groups, a potency test was carried out as described in the Example 1.3 above. The compositions of sEVs showing an average value exceeding 50% were considered as proangiogenic active.

The results of the angiogenic potency test were validated in vivo using proangiogenic effective and ineffective sEVs from different patient's groups (Figure 2A-B).

### 2.3 TGF$\beta$ content in sEVs and their angiogenic potential

To investigate whether the TGF$\beta$ content in sEVs may account for their angiogenic potential, the inventors carried out a ELISA assay on the EVs isolated from serum samples of healthy subjects and patients (diabetic, obese, diabetic/obese and ischemic patients). As shown in Figure 3, the content of TGF$\beta$ measured in the sEVs compositions correlates significantly with the proangiogenic potential of these vesicles in patient cohorts as well as in healthy donors. A cut-off value corresponding to a concentration of TGF$\beta$ of 23 pg/$10^{10}$ EVs was determined that discriminates proangiogenic effective EVs from ineffective vesicles based on the observation that EVs having a TGF$\beta$ content < 23 pg/$10^{10}$ EVs are more likely to be proangiogenic inactive.

### 2.4 miRNome profile of sEVs

The miRNome analysis carried out by the present inventors on proangiogenic effective and ineffective sEVs from healthy donors (3 samples/each) led to the identification of eight angio-miRNAs as the most differentially expressed, miR-126, miR-21, miR-296-3p, miR-210, miR-130a, miR-27a, miR-29a, miR-191. In particular, miR-126, miR-130a, miR-27a and 296-3p were up-regulated, while miR-21, miR-29a, miR-191 and miR-210 were downregulated in sEVs with proangiogenic capability.

To investigate whether the observed difference in miRNAs expression levels in EVs is associated with their functional activity, the inventors carried out a study by comparing the expression of selected miRNAs in sEVs derived from individual healthy donors and patients, with the level of proangiogenic activity of these vesicles as measured with the in vitro potency test. The expression analysis was performed by real time PCR (cut-off Ct value 30). As shown in Figure 4A, the distribution of the Ct values measured for miR-130a in the EVs from individual subjects (healthy donors and patients) correlates significantly with the results of the angiogenesis potency test performed on these EVs samples. Particularly, it was observed that EVs having a content of miR130a measured as Ct value of Ct > 30 have a higher probability of being proangiogenic ineffective.

Interestingly, the present inventors found also an enrichment of miR-210 in sEVs derived from patients. as previous described in Shalaby SM. et al, "Serum miRNA-499 and miRNA-210: A potential role in early diagnosis of acute coronary syndrome" IUBMB Life. 2016;68(8):673-82. However, no significant correlation was detected between miR-210 content in sEVs and the proangiogenic activity of these vesicles.

DIANA mirpath analysis was interrogated using miR-130a by selecting pathways involving at least 15 genes. Again, among others, a significant enrichment of genes involved in the TGFβ pathway was detected (Figure 4B).

Network predicted by IPA for miR-130a target genes identified several genes, such as KDR, HOXA5, ROCK1, EPHB6, strongly related to the angiogenic process (Figure 5A). Moreover, TGFβ and TGFBR1 genes were found among the miR-130a interactors. Overall, the above described results further support the contribution of the TGFβ signaling pathway in sEV-mediated mechanisms of action.

### 2.5 The content of miR-130a and TGFβ in sEVs represents a valuable predictive marker to identify "true proangiogenic ineffective" sEVs.

The inventors carried out a Receiver Operator Characteristic (ROC) analysis to assess whether the content of miR-130a and TGFβ in sEVs has the predictive capacity to discriminate between sEVs displaying proangiogenic capability and ineffective vesicles. As deduced from the ROC curves illustrated in Figure 5B, both miR-130a and TGFβ are good predictive measures of "true proangiogenic ineffective sEVs" identified by RS, showing statistically significant AUC values.

Both measures displayed a good sensitivity to identify as "proangiogenic ineffective" the "true proangiogenic ineffective sEVs" identified by RS. This was particularly evident and further underlined by the LH + = 1.88 IC 95% from 1.49 to 2.27, for miR-130a (Se = 0.94 IC95% from 0.73 to 0.99) and for TGFβ (Se = 0.88 IC95% from 0.66 to 0.97). However, a low specificity value for both measurements was detected (miR-130a: Sp = 0.50; TGFβ Sp = 0.64).

By combining the two measures 'in Series', i.e. considering as "proangiogenic ineffective" those sEVs defined as "proangiogenic ineffective" in both measures, a good level of sensitivity and an increased specificity value were detected (Sp= 0.75; Se= 0.82). The LH + value reported in Table 1 below further supports these results.

**Table 1**

| Test combining miR-130a and TGFβ1 'in series'. List of values obtained combining the two measures 'in Series' (considering as "proangiogenic ineffective" the sEVs defined as "proangiogenic ineffective" in both miR-130a and TGFβ1 measures). | | | |
|---|---|---|---|
| | | **95% Conf. Int.** | |
| *Parameters* | | *Inf* | *Sup* |
| *Se* | 0.824 | 0.59 | 0.94 |
| *Sp* | 0.750 | 0.57 | 0.87 |
| *ACC* | 0.778 | 0.64 | 0.87 |
| *VPP* | 0.667 | 0.45 | 0.83 |
| *VPN* | 0.875 | 0.69 | 0.96 |
| *LH+* | 3294118 | 2.62 | 3.97 |
| *LH-* | 0.235294 | -0.81 | 1.28 |

### Claims

1. A method of predicting whether a composition of extracellular vesicles (EVs) has proangiogenic activity, comprising the steps of:

   (a) quantifying the miR-130a microRNA content in the composition of EVs, and
   (b) quantifying the transforming growth factor beta (TGFβ) content in the composition of EVs;
   (c) determining whether the miR-130a content is above a first predetermined value and the TGFβ content is above a second predetermined value,

wherein:
when the miR-130a content is above said first predetermined value and the TGFβ content is above said second predetermined value, the composition of EVs is predicted to have proangiogenic activity.

2. The method according to claim 1, wherein the miR-130a content is quantified as a Ct value by real-time polymerase chain reaction (real-time PCR) and wherein there is an inverse correlation between the miR-130a content and the Ct value.

3. The method according to claim 2, wherein said first predetermined value is a Ct value < 30.

4. The method according to any of claims 1 to 3, wherein the TGFβ content is measured by an immunoassay, preferably by a enzyme-linked immunosorbent assay (ELISA).

5. The method according to any of claims 1 to 4, wherein said second predetermined value is an amount of TGFβ of 23 pg/$10^{10}$ EVs.

6. The method according to any of claims 1 to 5, further comprising step (d) of quantifying the proangiogenic activity of the EVs composition by means of a potency test, said potency test comprising the steps of:

   - testing the composition of EVs by a BrdU cell proliferation assay to obtain a composition value;
   - testing a negative control by a BrdU cell proliferation assay to obtain a negative control value;
   - testing a positive control by a BrdU cell proliferation assay to obtain a positive control value;
   - calculating the % proangiogenic activity of the composition of EVs in the BrdU cell proliferation assay by applying the following formula:

$$\% \, proangiogenic \, activity = \frac{composition \, value - negative \, control \, value}{positive \, crtl \, value - negative \, control \, value} \, x \, 100;$$

   and/or
   said potency test comprising the steps of:

   - testing the composition of EVs by a tubulogenesis assay to obtain a composition value;
   - testing a negative control by a tubulogenesis assay to obtain a negative control value;
   - testing a positive control by a tubulogenesis assay to obtain a positive control value;
   - calculating the % proangiogenic activity of the composition of EVs in the tubulogenesis assay by applying the following formula:

$$\% \, proangiogenic \, activity = \frac{composition \, value - negative \, control \, value}{positive \, crtl \, value - negative \, control \, value} \, x \, 100.$$

7. The method according to any of claim 1 to 6, wherein the EVs are from human cells.

8. A method of manufacturing a preparation of proangiogenic extracellular vesicles (EVs), comprising the steps of:

   - isolating EVs from multiple preparations of a body fluid or from the conditioned medium of a cell culture;
   - preparing one or more samples from the isolated EVs at a predetermined concentration of EVs;
   - predicting the proangiogenic activity of each EVs sample with a method according to any of claims 1 to 7;
   - selecting the samples wherein miR-130a content is above said first predetermined value, and TGFβ is above said second predetermined value; and optionally
   - pooling two or more of the active EVs samples,

   thereby obtaining a preparation of proangiogenic EVs.

9. The method according to claim 8, wherein the EVs preparation is determined to have a proangiogenic activity of at least 50%.

10. A preparation of proangiogenic extracellular vesicles (EVs) having a miR-130a content measured as Ct value by real-time PCR of Ct < 30 and/or a TGFβ content > 23 pg/$10^{10}$ EVs, for use in the therapeutic treatment of a disease or injury positively influenced by proangiogenic therapy or for use in wound healing.

11. The preparation for use according to claim 10, wherein the EVs preparation has a proangiogenic activity of at least 50%.

12. The preparation for use according to claim 10 or 11, wherein the EVs are derived from a biological fluid or from a conditioned cell or tissue culture medium, wherein the biological fluid is whole blood, plasma, or serum.

13. The preparation for use according to claim 12, wherein the EVs are prepared from serum of a healthy donor or from serum of a patient with cardiovascular risk factors.

14. The preparation for use according to any of claims 10 to 13, wherein the disease or injury is a vascular disease or injury, or a condition associated with risk of cardiovascular disease.

15. The preparation for use according to any of claims 10 to 14, wherein the disease or injury is selected from the group consisting of acute myocardial infarction, acute cerebrovascular disease, acute and chronic peripheral artery disease, acute kidney ischemia, obesity and diabetes mellitus.

**Patentansprüche**

1. Verfahren zur Vorhersage, ob eine Zusammensetzung extrazellulärer Vesikel (EV) eine proangiogene Aktivität aufweist, umfassend die folgenden Schritte:

(a) Quantifizieren des miR-130a MikroRNA Gehalts in der Zusammensetzung der EV; und
(b) Quantifizieren des Gehalts an transformierendem Wachstumsfaktor Beta (TGFβ) in der Zusammensetzung der EV;
(c) Bestimmen, ob der miR-130a-Gehalt über einem ersten vorbestimmten Wert liegt und der TGFβ-Gehalt über einem zweiten vorbestimmten Wert liegt,

wobei:
wenn der miR-130a-Gehalt über dem ersten vorbestimmten Wert liegt und der TGFβ-Gehalt über dem zweiten vorbestimmten Wert liegt, wird man vorhergesagt, dass die EV-Zusammensetzung eine proangiogene Aktivität aufweist.

2. Verfahren nach Anspruch 1, wobei der miR-130a-Gehalt als Ct-Wert mittels Echtzeit-Polymerase-Kettenreaktion (Echtzeit-PCR) quantifiziert wird und wobei eine inverse Korrelation zwischen dem miR-130a-Gehalt und dem Ct-Wert vorhanden ist.

3. Verfahren nach Anspruch 2, wobei der erste vorbestimmte Wert ein Ct-Wert < 30 ist.

4. Verfahren nach einem der Ansprüche 1 bis 3, wobei der TGFβ-Gehalt durch einen Immunoassay, vorzugsweise durch einen Enzymgekoppelter Immunabsorptionsassay (ELISA), gemessen wird.

5. Verfahren nach einem der Ansprüche 1 bis 4, wobei der zweite vorbestimmte Wert eine TGFβ-Menge von 23 pg/$10^{10}$ EVs ist.

6. Verfahren nach einem der Ansprüche 1 bis 5, ferner umfassend den Schritt (d) des Quantifizierens der proangiogenen Aktivität der EV-Zusammensetzung mittels eines Zustandstests, wobei der Zustandstest die folgenden Schritte umfasst:

- Testen der EV-Zusammensetzung mittels eines BrdU-Zellproliferationsassays, um einen Zusammensetzungswert zu erreichen;
- Testen einer negativen Kontrolle mittels eines BrdU-Zellproliferationsassays, um einen negativen Kontrollwert zu erreichen;

- Testen einer positiven Kontrolle mittels eines BrdU-Zellproliferationsassays, um einen positiven Kontrollwert zu erreichen;

- Berechnen des proangiogenen Aktivitätsprozentsatzes der EV-Zusammensetzung in dem BrdU-Zellproliferationsassay nach der folgenden Formel:

$$\% \ proangiogene \ Aktivitat = \frac{Zusammensetzungswert - negativer \ Kontrollwert}{positiver \ crtI \ Wert - negativer \ Kontrollwert} \quad X \quad 100$$

und/oder

der Zustandstest umfasst die folgenden Schritte:

- Testen der EV-Zusammensetzung mittels eines Tubulogenesisassay, um einen Zusammensetzungswert zu erreichen;

- Testen einer negativen Kontrolle mittels eines Tubulogenesisassay, um einen negativen Kontrollwert zu erreichen;

- Testen einer positiven Kontrolle mittels eines Tubulogenesisassay, um einen positiven Kontrollwert zu erreichen;

- Berechnen des proangiogenen Aktivitätsprozentsatzes der EV-Zusammensetzung in dem Tubulogenesisassay nach der folgenden Formel:

$$\% \ proangiogene \ Aktivitat = \frac{Zusammensetzungswert - negativer \ Kontrollwert}{positiver \ crtl \ Wert - negativer \ Kontrollwert} X \ 100$$

7. Verfahren nach einem der Ansprüche 1 bis 6, wobei die EV menschliche Zellen sind.

8. Verfahren zum Herstellen einer Zubereitung proangiogener extrazellulärer Vesikel (EV), umfassend die folgenden Schritte:

- Isolieren EV aus mehreren Zubereitungen einer Körperflüssigkeit oder aus dem konditionierten Medium einer Zellkultur;
- Vorbereiten einer oder mehrerer Proben aus den isolierten EV bei einer vorbestimmten Konzentration von EV;
- Vorhersagen der proangiogenen Aktivität jeder EV-Probe mittels eines Verfahrens nach einem der Ansprüche 1 bis 7;
- Auswahlen der Proben, bei denen der miR-130a-Gehalt über dem ersten vorbestimmten Wert liegt und der TGFβ-Gehalt über dem zweiten vorbestimmten Wert liegt; und optional
- Vereinigen zwei oder mehrere der aktiven EV-Proben,

wodurch eine Zubereitung proangiogener EV erreicht wird.

9. Verfahren nach Anspruch 8, wobei die EV-Zubereitung eine proangiogene Aktivität von mindestens 50 % aufweist.

10. Zubereitung proangiogener extrazellulärer Vesikel (EV) mit einem miR-130a-Gehalt, gemessen als Ct-Wert mittels Echtzeit-PCR von Ct < 30 und/oder einem TGFβ-Gehalt > 23 pg/$10^{10}$ EV, zum Verwenden in der therapeutischen Behandlung einer Erkrankung oder Verletzung, die positiv durch eine proangiogene Therapie beeinflusst wird, oder zum Verwenden in der Wundheilung.

11. Zubereitung zum Verwenden nach Anspruch 10, wobei die EV-Zubereitung eine proangiogene Aktivität von mindestens 50 % aufweist.

12. Zubereitung zum Verwenden nach Anspruch 10 oder 11, wobei die EV aus einer biologischen Flüssigkeit oder aus einem konditionierten Zell- oder Gewebekulturmedium gewonnen werden, wobei die biologische Flüssigkeit Vollblut, Plasma oder Serum ist.

13. Zubereitung zum Verwenden nach Anspruch 12, wobei die EV aus dem Serum eines gesunden Spenders oder aus dem Serum eines Patienten mit kardiovaskulären Risikofaktoren vorbereitet werden.

**14.** Zubereitung zum Verwenden nach einem der Ansprüche 10 bis 13, wobei die Erkrankung oder die Verletzung eine vaskuläre Erkrankung oder Verletzung ist oder einen Zustand ist, der mit dem Risiko einer kardiovaskulären Erkrankung assoziiert ist.

**15.** Zubereitung zum Verwenden nach einem der Ansprüche 10 bis 14, wobei die Erkrankung oder die Verletzung aus der Gruppe bestehend aus akutem Myokardinfarkt, akuter zerebrovaskulärer Erkrankung, akuter und chronischer peripherer arterieller Erkrankung, akuter Nierenischämie, Adipositas und Diabetes mellitus ausgewählt wird.

**Revendications**

**1.** Un procédé de prédiction de l'activité proangiogénique d'une composition de vésicules extracellulaires (EV), comprenant les étapes de:

> (a) quantifier la teneur en microARN miR-130a dans la composition des EV; et
> (b) quantifier la teneur en facteur de croissance transformant bêta (TGFβ) dans la composition des EV;
> (c) déterminer si la teneur en miR-130a est supérieure à une première valeur prédéterminée et si la teneur en TGFβ est supérieure à une deuxième valeur prédéterminée,

dans lequel:
lorsque la teneur en miR-130a est supérieure à ladite première valeur prédéterminée et la teneur en TGFβ est supérieure à ladite deuxième valeur prédéterminée, la composition des EV est prédite comme ayant une activité proangiogénique.

**2.** Le procédé selon la revendication 1, dans lequel la teneur en miR-130a est quantifiée sous forme d'une valeur Ct par réaction en chaîne par polymérase en temps réel (PCR en temps réel) et dans lequel il existe une corrélation inverse entre la teneur en miR-130a et la valeur Ct.

**3.** Le procédé selon la revendication 2, dans lequel ladite première valeur prédéterminée est une valeur Ct < 30.

**4.** Le procédé selon l'une quelconque des revendications 1 à 3, dans lequel la teneur en TGFβ est mesurée par un immunodosage, de préférence par un test immuno-enzymatique (ELISA).

**5.** Le procédé selon l'une quelconque des revendications 1 à 4, dans lequel ladite deuxième valeur prédéterminée est une quantité de TGFβ de 23 pg/$10^{10}$ EV.

**6.** Le procédé selon l'une quelconque des revendications 1 à 5, comprenant en outre l'étape (d) de quantification de l'activité proangiogénique de la composition des EV au moyen d'un test de puissance, ledit test de puissance comprenant les étapes de:

> - tester la composition des EV par un test de prolifération cellulaire BrdU pour obtenir une valeur de composition;
> - tester un témoin négatif par un test de prolifération cellulaire BrdU pour obtenir une valeur de témoin négatif;
> - tester un témoin positif par un test de prolifération cellulaire BrdU pour obtenir une valeur de témoin positif;
> - calculer le pourcentage d'activité proangiogénique de la composition des EV dans le test de prolifération cellulaire BrdU en appliquant la formule suivante:

$$\% \; activité \; proangiogenique = \frac{valeur \; de \; composition - valeur \; de \; temoin \; negatif}{valeur \; de \; temoin \; positif - valeur \; de \; temoin \; negatif} \; X \; 100$$

et/où
- ledit test de puissance comprenant également les étapes de:

> - tester la composition des EV par un test de tubulogenèse pour obtenir une valeur de composition;
> - tester un témoin négatif par un test de tubulogenèse pour obtenir une valeur de témoin négatif;
> - tester un témoin positif par un test de tubulogenèse pour obtenir une valeur de témoin positif;
> - calculer le pourcentage d'activité proangiogénique de la composition des EV dans le test de tubulogenèse en appliquant la formule suivante:

$$\% \; activité \; proangiogenique = \frac{valeur \; de \; composition - valeur \; de \; temoin \; negatif}{valeur \; de \; temoin \; positif - valeur \; de \; temoin \; negatif} \; X \; 100$$

7.  Le procédé selon l'une quelconque des revendications 1 à 6, dans lequel les EV proviennent de cellules humaines.

8.  Un procédé de fabrication d'une préparation de vésicules extracellulaires (EV) proangiogéniques, comprenant les étapes de:

    - isoler des EV à partir de plusieurs préparations d'un fluide corporel ou du milieu conditionné d'une culture cellulaire;
    - préparer un ou plusieurs échantillons à partir des EV isolées à une concentration prédéterminée des EV;
    - prédire l'activité proangiogénique de chaque échantillon des EV à l'aide d'un procédé selon l'une quelconque des revendications 1 à 7;
    - sélectionner les échantillons dans lesquels la teneur en miR-130a est supérieure à ladite première valeur prédéterminée et la teneur en TGFβ est supérieure à ladite deuxième valeur prédéterminée; et, éventuellement,
    - regrouper deux ou plusieurs des échantillons des EV actifs,

    permettant ainsi d'obtenir une préparation des EV proangiogéniques.

9.  Le procédé selon la revendication 8, dans lequel la préparation des EV est déterminée comme ayant une activité proangiogénique d'au moins 50 %.

10. Une préparation de vésicules extracellulaires (EV) proangiogéniques ayant une teneur en miR-130a mesurée sous forme de valeur Ct par PCR en temps réel de Ct < 30 et/ou une teneur en TGFβ > 23 pg/$10^{10}$ EV, destinée à être utilisée dans le traitement thérapeutique d'une maladie ou d'une lésion influencée positivement par une thérapie proangiogénique ou destinée à être utilisée dans la cicatrisation des plaies.

11. La préparation pour utilisation selon la revendication 10, dans laquelle la préparation des EV a une activité proangiogénique d'au moins 50 %.

12. La préparation pour utilisation selon la revendication 10 ou 11, dans laquelle les EV sont dérivées d'un fluide biologique ou d'un milieu conditionné de culture cellulaire ou tissulaire, le fluide biologique étant du sang total, du plasma ou du sérum.

13. La préparation pour utilisation selon la revendication 12, dans laquelle les EV sont préparées à partir du sérum d'un donneur sain ou du sérum d'un patient présentant des facteurs de risque cardiovasculaire.

14. La préparation pour utilisation selon l'une quelconque des revendications 10 à 13, dans laquelle la maladie ou la lésion est une maladie vasculaire ou une lésion vasculaire, ou une condition associée à un risque de maladie cardiovasculaire.

15. La préparation pour utilisation selon l'une quelconque des revendications 10 à 14, dans laquelle la maladie ou la lésion est sélectionnée dans le groupe constitué par l'infarctus aigu du myocarde, la maladie cérébrovasculaire aiguë, la maladie artérielle périphérique aiguë et chronique, l'ischémie rénale aiguë, l'obésité et le diabète sucré.

FIG.1

FIG.1

**A**

**B**

FIG.2

FIG.3

( continued )

FIG.3

FIG.4

FIG.4

**A**

**FIG.5**

**B**

| | | | | CI 95% | | | | | CI 95% |
|---|---|---|---|---|---|---|---|---|---|
| AUC | Std error | Signific. | Inf | Sup | AUC | Std error | Signific. | Inf | Sup |
| 0.761 | 0.071 | 0.004 | 0.621 | 0.9 | 0.765 | 0.07 | 0.03 | 0.627 | 0.903 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018069408 A

**Non-patent literature cited in the description**

- **PATHAN M et al.** Vesiclepedia 2019: a compendium of RNA, proteins, lipids and metabolites in extracellular vesicles. *Nucleic Acids Res.*, 2019, vol. 47, D516-D519
- **LIVAK KJ** ; **SCHMITTGEN TD**. Analysis of relative gene expression data using real- time quantitative PCR and the 2(- Delta Delta C(T)) method. *Methods*, 2001, vol. 25, 402-408
- **CAVALLARI C. et al.** Serum-derived extracellular vesicles (EVs) impact on vascular remodeling and prevent muscle damage in acute hind limb ischemia. *Sci Rep.*, 2017, vol. 7 (1), 8180
- **LOPATINA T. et al.** Platelet-derived growth factor regulates the secretion of extracellular vesicles by adipose mesenchymal stem cells and enhances their angiogenic potential. *Cell Commun Signal.*, 2014, vol. 12, 26
- **COLLINO F. et al.** Exosome and Microvesicle-Enriched Fractions Isolated from Mesenchymal Stem Cells by Gradient Separation Showed Different Molecular Signatures and Functions on Renal Tubular Epithelial Cells. *Stem Cell Rev.*, 2017, vol. 2, 226-43
- **GRUND B** ; **SABIN C**. Analysis of biomarker data: logs, odds ratios, and receiver operating characteristic curves. *Curr Opin HIV AIDS*, 2010, vol. 5 (6), 473-9
- **SHALABY SM et al.** Serum miRNA-499 and miRNA-210: A potential role in early diagnosis of acute coronary syndrome. *IUBMB Life.*, 2016, vol. 68 (8), 673-82